# EUROPEAN PATENT SPECIFICATION

(11) **EP 3 305 769 B1**
(45) Date of publication and mention of the grant of the patent: **26.06.2019**
(21) Application number: 17020458.0
(22) Date of filing: 06.10.2017
(51) Int. Cl.: C07D 217/26, C07D 407/10, C07D 263/34

(54) **METHOD FOR PREPARATION OF (7-PHENOXY-4-HYDROXY-1-METHYL-ISOQUINOLINE-3-CARBONYL)-GLYCINE (ROXEDUSTAT) AND ITS INTERMEDIATES BASED ON SIMULTANEOUS OPENING OF OXAZOLIC RING, FISSION OF ETHER AND CREATION OF IMINE**
VERFAHREN ZUR HERSTELLUNG VON (7-PHENOXY-4-HYDROXY-1-METHYL-ISOCHINOLIN-3-KARBONYL)GLYCIN (ROXADUSTAT) UND DESSEN ZWISCHENPRODUKTEN, BASIEREND AUF GLEICHZEITIGER OXAZOLRINGÖFFNUNG, ETHERSPALTUNG UND IMINBILDUNG
PROCÉDÉ DE PRÉPARATION DE (7-PHÉNOXY-4-HYDROXY-1-MÉTHYL-ISOQUINOLÉINE-3-CARBONYL)-GLYCINE (ROXADUSTAT) ET SES INTERMÉDIAIRES SUR LA BASE DE L'OUVERTURE SIMULTANÉE DU CYCLE OXAZOLIQUE, DE LA FISSION D'ÉTHER ET DE LA CRÉATION D'IMINE

(30) Priority: 07.10.2016 CZ 20160627
(43) Date of publication of application: 11.04.2018
(73) Proprietor: Zentiva K.S., 102 37 Praha 10 (CZ)
(72) Inventor: Pisa, Ondrej, 378 31 Horni Pena (CZ); Radl, Stanislav, 250 84 Kvetnice (CZ); Stach, Jan, 190 16 Praha 9 (CZ)
(74) Representative: Jirotkova, Ivana

(56) References cited:
- WO-A1-2004/108681
- WO-A1-2013/013609

## Description

### Field of the Invention

The invention is related to a new and efficient way of preparation of (7-phenoxy-4-hydroxy-1-methyl-isoquinoline-3-carbonyl)-glycine of formula **1**, that is known under generic name of roxadustat, and its intermediates.

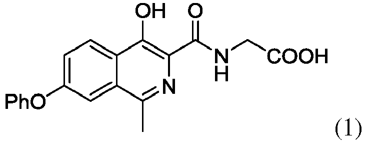

Roxadustat of formula **1** is orally administered inhibitor of Hypoxia-Inducible Factor (HIF) of prolyl-hydroxylase. It supports erythropoiesis by increasing the level of endogenous erythropoietin and so improves regulation of iron and decrease level of hepcidin. It is used for treatment of anemia at patients with chronic kidney disease.

### Background Art

Preparation of roxadustat of formula **1** was described in a patent application WO2004108681, where the roxadustat is prepared in 11 steps from initial chemical compound 4-nitro-1,2-dicarbonitrile of formula **2.** Reaction of dicarbonitrile of formula **2** with phenol and potash in the first step leads to preparation of corresponding phenoxy derivate of formula **3,** 4-phenoxyphthalic acid of formula 4 is prepared by the following hydrolysis effect of potassium hydroxide in methanol, from which a solid-phase mixture with glycine is created for reaction in a melt with temperature of 210°C to 220°C, which provides corresponding phthalimide of formula **5.** After that, phthalimide of formula **5** is esterified to corresponding methyl ester of formula **6.** Then, expansion of the ring is performed by the effect of sodium butanolate to butyl-7-phenoxy-1,4-dihydroxy isoquinoline-3-carboxylate of formula **7.** Hydroxyl group of derivative of formula **7** in a position 1 is then replaced by bromine using phosphorus oxybromide under the alkaline conditions resulting in creation of isoquinoline derivative of formula **8.** Further the alkaline hydrolysis is carried out to provide an acid of formula **9.** Then the lithiation is carried out, capture of lithium salt with methyl iodide, and in the second step, the hydroxyl group and carboxyl function are protected by benzyl group resulting in the derivative of formula **10.** By the effect of potassium hydroxide the derivative of formula **10** is hydrolyzed to the carboxylic acid of formula **11,** from which the derivative **12** is prepared under the conditions of glycine benzyl ester. The final step of synthesis of roxadustat of formula **1** is deprotection of benzyl groups by means of hydrogenation reaction (Diagram 1). The procedure is stated in examples of the invention without the corresponding diagrams and in most cases no yields are stated. The procedure of roxadustat preparation is stated only as an analogy to similar examples.

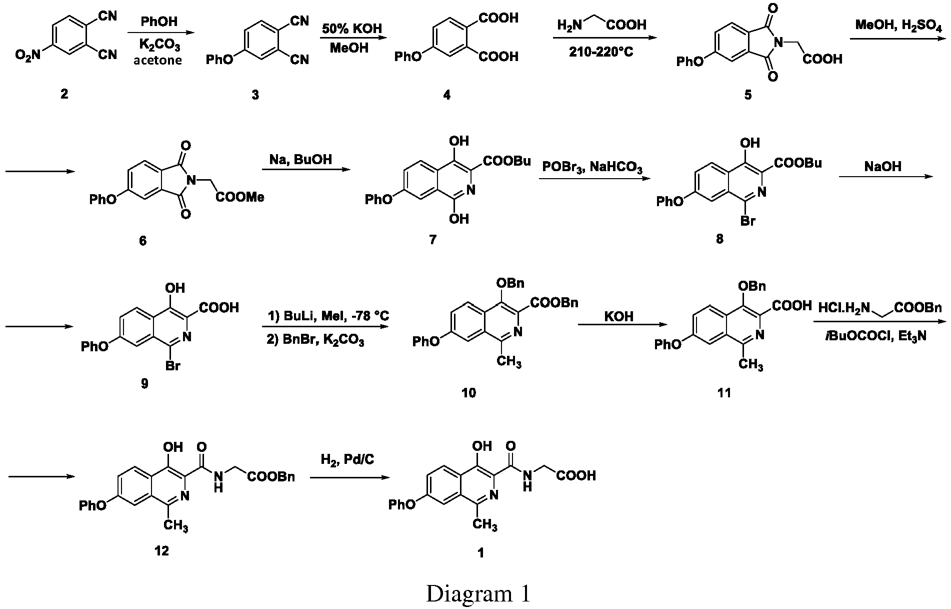

Patent application WO2013013609 describes a 10-step synthesis which is based, as well as the synthesis in the patent application WO2004108681, on using 4-nitrobenzene-1,2-dicarbonitrile of formula **2** which is converted using phenol and potash to 4-phenoxybenzene-1,2-dicarbonitrile of formula **3** with a yield of 99%. In the next step, hydrolysis to 4-phenoxyphthalic acid of formula **4** is carried out, which is transformed using acetic anhydride and acetic acid to phthalic anhydride derivative of formula **13,** which is converted to oxazole derivative of formula **14** (in quantitative yield) using methyl isocyanoacetate and DBU (1,8-diazabicyclo[5.4.0]undec-7-ene) in THF (tetrahydrofuran), in which then the effect of concentrated hydrochloric acid in methanol leads to opening of its oxazole ring and closing of isoquinoline ring while a derivative of formula **15** is performed (the mentioned yield is 22% only). The following reaction with phosphorus oxychloride provides a chloro-derivative of formula **16** which is converted to methyl-7-phenoxy-4-hydroxy-1-chloroquinoline-3-carboxylate of formula **17** in yield of 33%. The ester of formula **17** undergoes to alkaline hydrolysis to produce the corresponding acid of formula **18** which is under specific conditions with methylglycinate hydrochloride, benzotriazol-1-yl-oxytripyrrolidinophosphonium hexafluorophosphate (PyBOP), bis(isopropyl)ethylamine and triethylamine (TEA) transferred to ester of formula **19,** which hydrolysis in the final step leads to preparation of roxadustat of formula **1** in yield of 78% (Diagram 2). The procedure is mentioned in the patent application including diagrams and most reaction also contains yields.

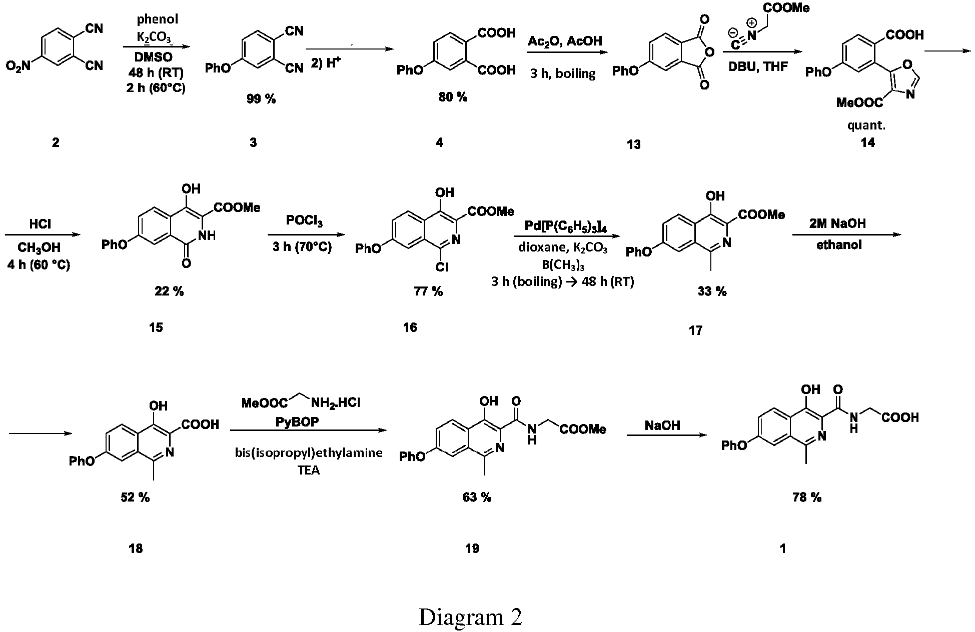

Patent file WO2014014834 related to the preparation of isoquinoline compounds describes a 9-step synthesis which is based on 5-bromophthalide of formula **20** which is converted to a phenoxy derivative of formula **21** using Ullman type reaction. 5-phenoxyphthalide of formula **21** is subsequently opened under the specific conditions to a chloride of acid of formula **21a** which decomposes with methanol to the methyl ester of formula **22.** Substitution of the chloride at the benzyl position with the use of tosylated glycine methyl ester (Ts-Gly-OMe) is then carried out under the conditions of the Finkelstein reaction to form an intermediate of formula **22a,** in which the isoquinoline ring is closed with the effect of sodium methoxide and subsequent acid decomposition to form methyl 7-phenoxy-4-hydroxyisoquinoline-3-carboxylate of formula **23.** The isoquinoline derivative of formula **23** is substituted by the effect of dimethylamino methane in acetic acid at position 1 to the dimethylaminomethyl derivative of formula **24** which is used without isolation into the subsequent reaction with the acetic anhydride with the aim of replace the dimethylamino group with the acetoxy group. In this step undesirable protection of the hydroxyl group may occur by the acetyl group, however this can be solved by the addition of morpholine, which will ensure the transformation of the diacetylated derivative of formula **25b** back into the monoacetylated derivative of formula **25a.** By hydrogenation reaction, the monoacetylated derivative of formula **25a** is converted to the methyl ester of formula **17,** which is then converted to the final roxadustat of formula 1 by the effect of glycine and of the sodium methanolate solution in methanol in a pressure vessel at 110°C and by subsequent acidification, see Diagram 3.

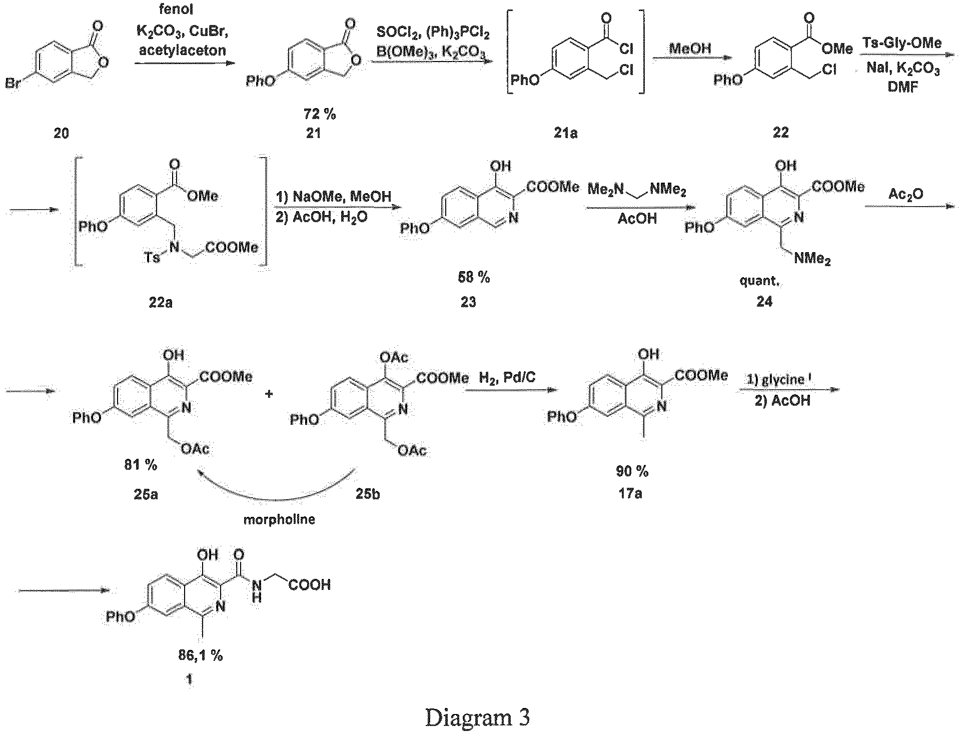

The patent application CN104892509 describes a 6-step synthesis based on L-tyrosine of formula **26,** which is converted to the corresponding ester of general formula **27.** The ester of formula 27 is then substituted with the Ulmann type reaction on the hydroxyl group by phenyl, to form the corresponding tyrosine derivative of formula 28. The Pictet-Spengler process with acetaldehyde in an acidic medium then gives the product of cyclization of formula **29,** which is oxidized to 7-phenoxy-1-methylisoquinoline-3-carboxylate of general formula **30.** Oxidation by the effect of hydrogen peroxide under the specific conditions (Ac2O and p-TsCl, see Tetrahedron 1963, 19, 827-832) gives a hydroxy derivative of formula 17, which in the last step is converted to roxadustat of formula 1 by the reaction with glycine in a solution of sodium methoxide in methanol in a pressure vessel at 110°C (Diagram 4).

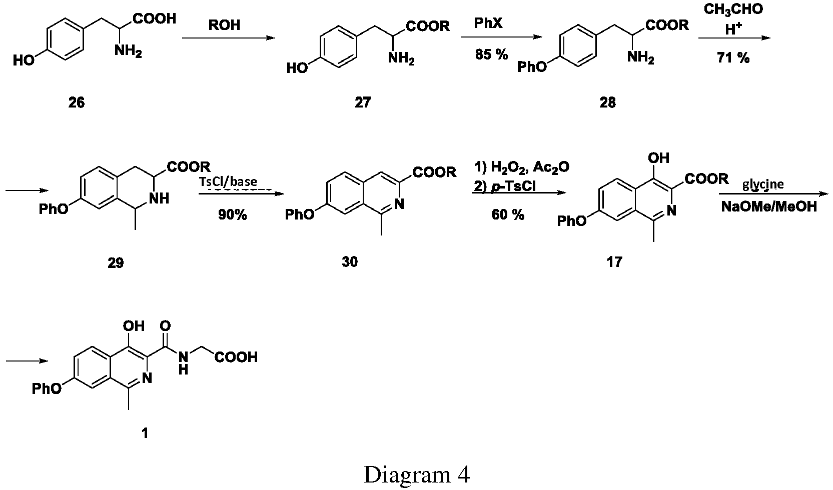

### Disclosure of the Invention

The invention relates to a new effective process for a preparation of (7-phenoxy-4-hydroxy-1-methylisoquinoline-3-carbonyl) glycine of formula **1** known under the generic name roxadustat, which is based on the synthesis of an intermediate of formula **31,** wherein X = I, Br or Cl, or other groups suitable for coupling reactions such as OTf (Tf = triflate), R¹ is halogen, PhO (Ph = phenyl), OH or a protected hydroxyl group, R² is H or C1-C10 alkyl group and R³ is H or a C1-C10 alkyl group or aryl group, its conversion to a corresponding intermediate of formula **32,** , wherein R¹ is halogen, PhO, OH or protected hydroxyl group, substitute R² may be H or C1-C10 alkyl group, substitute R³ may be H or C1-C10 alkyl group or aryl group and group A is a precursor of the acetyl group, i.e., any functional group which may provide an acetyl group under the compatible conditions for opening of the oxazole ring. Possible examples of such group A include the group A1, wherein R⁴ can be C1-C10 alkyl group, group A2, wherein n = 1 to 5, A3, where a protective group PG can be SiR⁵R⁶R⁷ or CR⁸R⁹OH, wherein R⁵ to R⁹ can be C1-C10 alkyl group, or group A4. Group A can be directly the acetyl on its own. Intermediate of formula **32** can be converted under the conditions of this invention to non-isolated intermediate of formula **33,** that spontaneously cyclizes to key intermediate of formula **34,** wherein R¹ and R³ have the same meaning as at the compounds **31-33** (Diagram 5).

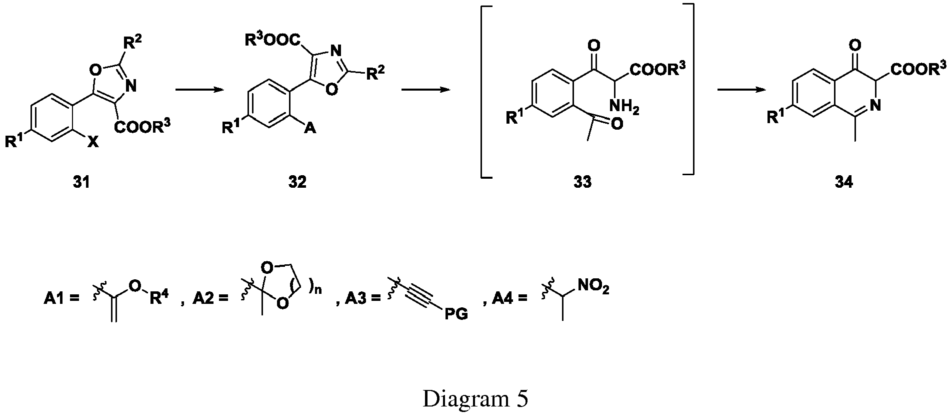

For the synthesis of roxadustat of formula **1** using this entirely new procedure there is no precedent in the literature or patent literature. This is in particular the preparation of the isoquinoline skeleton of formula **34,** which, unlike the prior art processes, in which it is prepared sequentially in the several reaction steps according to the invention can be carried out in one-step by use of suitable groupings that all react in acidic environment. In an acidic environment, the oxazole ring is cleaved to the corresponding amino acid ester and simultaneously under the appropriate conditions a transformation of the group A to the acetyl group occurs to form the compound of formula 33, and then to spontaneous cyclization to the isoquinoline skeleton of formula 34, which is essentially the corresponding imine, for which basically two tautomeric forms exist.

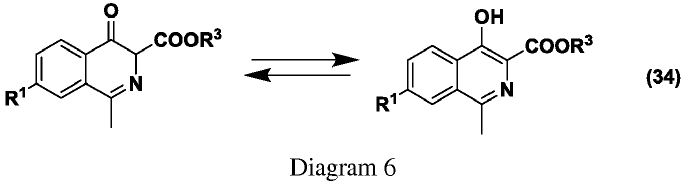

The corresponding compounds of formula **32** containing groups A1 can be obtained by Heck's coupling of suitable alkyl vinyl ethers with intermediates of formula **31.** In the literature, a number of alkyl vinyl ethers of formula **35** are used for this type of reaction, namely the compounds **35a-d** (Chem. Eur. J. 2008, 14, 5555). Since the alkyl group of the alkyl vinyl ether is not part of the target molecule in the present invention, this list is not limitative in terms of the subject of the invention.

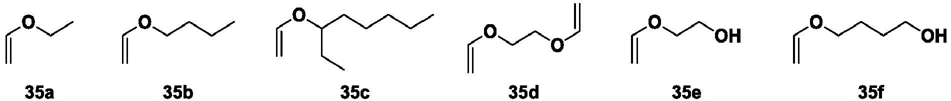

When a hydroxyl alkyl vinyl ether is used for Heck's coupling (e.g., compounds 35e or 35f), at a suitable arrangement the product is a compound of formula 32 having in the molecule an acetyl group A2 precursor

The corresponding substances **32** containing the groups A3 can be obtained by Sonogashirov's coupling of the appropriate acetylene derivatives **36** with intermediates **31.** In the literature, trialkylsilylacetylenes **36a-d** are used for this type of reaction, preferably trimethylsilylacetylene (**36a**) or 2,2-dialkyl derivatives of prop- 2-yn-1-ol **36e-g,** preferably 2-methylbut-3-yn-2-ol (**36c**).

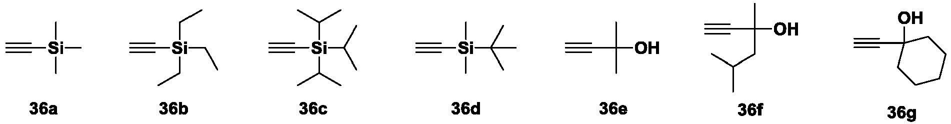

The corresponding compounds of formula **32** containing groups A4 can be obtained from the corresponding intermediates of formula **31** with Pd-catalyzed coupling with nitroethane (*J. Org. Chem.* **2002,** *67,* 106; *Tetrahedron* **2014,** *70,* 6384).

### Detailed description of the Invention

In principle, intermediates of formula **31** having phenoxy group or groups, that may be converted to phenoxy group in following steps (halogen, OH, or protected hydroxyl group, boronic acids, borates, etc.) as an R¹ can be used for the synthesis of roxadustat of formula **1.** Within the development of the method of synthesis of roxadustat of formula 1 we have verified that it is preferable to first introduce the phenoxy group into the molecule and therefore use the compounds **37** as a starting material. Literature does not mention the conversion of substances of formula **37** to the corresponding acid **38.** Its preparation was carried out by nucleophilic aromatic substitution. It is known from the literature that a similar result is theoretically achievable, for example, by using Ullmann's reaction, diazotation or coupling, when for example the groups selected from the range of -NO₂, N₂⁺, X, B(OH)₂, B(OR)₂ are a substitute for LG (Leaving Group). With regard to a commercial availability of substrates, ease of handling and subsequent reaction, the leaving group (LG) was chosen as F and X is any halide from the range of C1, Br, I or OTf. Since the hydrolysis to the corresponding carboxylic acid of formula **38,** which is advantageous to obtain the pure product, is carried out without isolation of the corresponding ester, the R group can be arbitrarily selected from the range of alkoxy, aryloxy, NH₂, NHR, NR₂, see Diagram 7. For the preparation of the oxazole ring in the substance of formula **31,** the reaction available from the literature was used, similarly used in patent application WO2013013609 for the preparation of the compound of formula **14.** It is a cyclization by the effect of isocyanoacetate, when the oxazole derivatives having a hydrogen atom as R² are formed. It is possible to use isocyanoacetates can be used, in which the substituent R³ can be selected from the range of H, C1 -C10 alkyl or aryl substituents, but due to a commercial availability, methyl- or ethyl ester are preferably used. The derivatives of formula **31** thus created are for simplicity hereinafter referred to as intermediates **39.** To ensure sufficient reactivity, it is first necessary to convert the carboxyl function of the intermediate of formula **38** to a suitable reactive analogue, for example by the effect of CDI (1,1'-carbonyldiimidazole), thionyl chloride, oxalyl chloride and other agents. The reaction provides a preparative yield of a compound of formula **39** (Example 2) of high purity as a white crystalline substance.

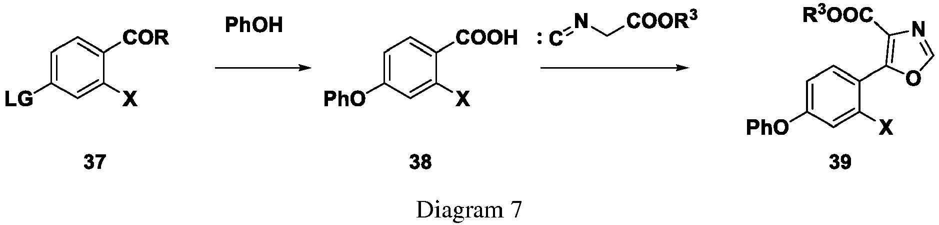

The essential step for the preparation of precursors for the preparation of the isoquinoline skeleton of formula **17** is the introduction of a suitable group, which can be converted into the corresponding acetyl in an acidic medium, to provide a one-step arrangement for the preparation of the isoquinoline skeleton of formula **17.** This condition is met by substances of structure **32** (Diagram 5), wherein acetyl group precursor is marked as group A. As a possible examples of such group A we indicate group A1, wherein R⁴ can be C1-C10 alkyl group, group A2, wherein n = 1 to 5, A3, wherein the protecting group PG can be SiR⁵R⁶R⁷ or CR⁸R⁹OH, wherein R⁵ to R⁹ can be C1-C10 alkyl group, or group A4. Intermediate of formula **32,** having phenoxy group as R¹ and H as R² is for simplicity hereinafter referred to as a substance of **formula 40.** Under the conditions of the invention, these intermediates of formulas **40-A1, 40-A2, 40-A3 a 40-A4** can be easily converted to a non-isolated intermediate of formula **41** (corresponds to structure **33,** wherein R¹ = PhO), which spontaneously cyclizes to the key intermediate of formula **17** (corresponds to the structure of formula **34** wherein R¹ = PhO), wherein R³ has the same meaning as for compounds of formulas **39** and **40** (Diagram 8 and 9). All the mentioned reactions are carried out best with substrates wherein X = Br, I, OTf. In the case of Heck's coupling for the preparation of compounds of general formula **40-A1,** the R⁴ group can be selected as arbitrary alkyl, it is possible to preferably use commercially available enol ethers **35a-d.** When the Heck coupling is carried out with substances that contain hydroxyl group in the alkyl chain, e.g., with enol ethers **35e, f,** the intermediates **40-A2** can be obtained *(*Chem. Eur. J. 2008, 14, 5555). To obtain the intermediates **40-A3,** the use of Sonogashir's coupling catalyzed by Pd catalysts using Cu cocatalyst (Synthesis 2011, 3604) or without Cu cocatalyst (J. Org. Chem. 2004, 69, 5428) is most preferable, and the use of a compound of formula **36** as an acetylene component, preferably the use of a substance **36a-d** with respect to the subsequent deprotection, the most preferably the use of trimethylsilylacetylene **36a.** Intermediate of type **40-A4** can then be obtained by Pd-catalyzed coupling with nitroethane (EtNO₂) (J. Org. Chem. 2002, 67, 106; Tetrahedron 2014, 70, 6384).

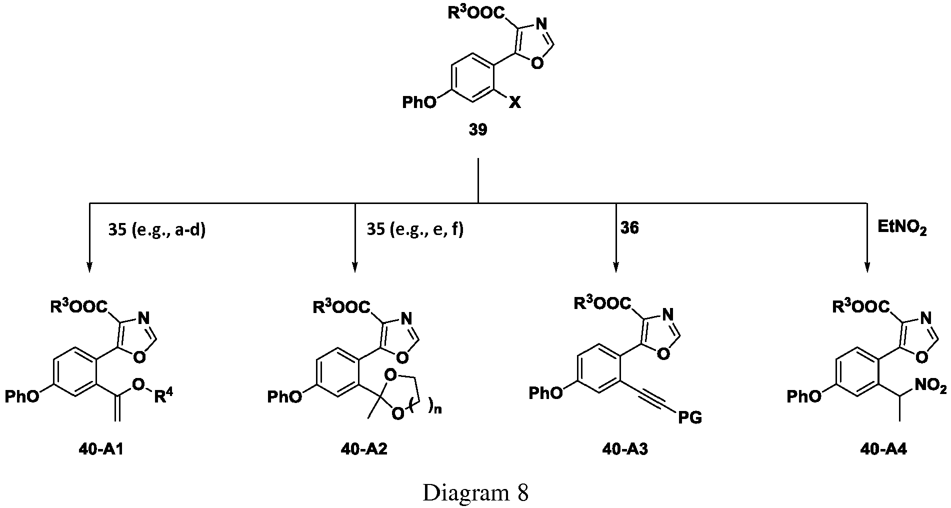

Precursors of formulas **40-A1, 40-A2, 40-A3** or **40-A4** for preparation of the isoquinoline skeleton of formula **17** are then converted to a non-isolated intermediate **41** (it is a substance of general formula **33** having R¹ = PhO). The conditions of this reaction were chosen with respect to the character of the A1-A4 groups, for the successful formation of the intermediate of formula **41** it was important to carry out the reaction in an acidic environment.

Intermediates of formulas **40-A1** and **40-A2** are preferably heated in an alcohol / concentrated HCl mixture, in which the acetyl group is gradually released from the enol ether grouping. In an acidic environment, an oxazole ring is also opened to form the aminoketoester. The substances in the reaction mixture are identified by mass spectrometry, so it is not possible to say in which order the steps are taking place. When both groupings are released from the precursors, the final formation of the imine, which is commonly catalyzed by acid, is allowed, and in this case the desired imine is the isoquinoline skeleton of formula **17.** The reaction mixture is processed by addition of water amount, extracted into a suitable organic solvent, boiled with activated charcoal, and the evaporation residue is then crystallized from the suitable alcohol to give white crystals of the compound of formula **17.**

In the case of intermediates 40-A3, the acetylene group must be converted to acetyl group to carry out the Markovnikov's water addition. Typically, this reaction is carried out by catalysis with various metallic compounds, but processes, which do not require such catalysis, are known as well. For our purposes we were able to apply a process using trifluoromethanesulfonic acid in trifluoroethanol (Org. Lett. 2016, 18, 2184). The reaction proceeds even in no presence of F⁻, however, it is more preferable to carry out desilylation with suitable fluorides (tetraalkylammonium fluorides, NaF, KF) before hydrolysis, preferably using tetrabutylammonium fluoride or KF, to create the intermediate **40-42.**

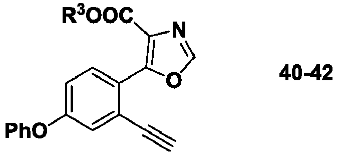

Intermediate **40-A4** can then be converted to the corresponding non-isolated intermediate **41** and subsequently to the isolated intermediate **17** in the similar way as substances **40-A1** a **40-A2.**

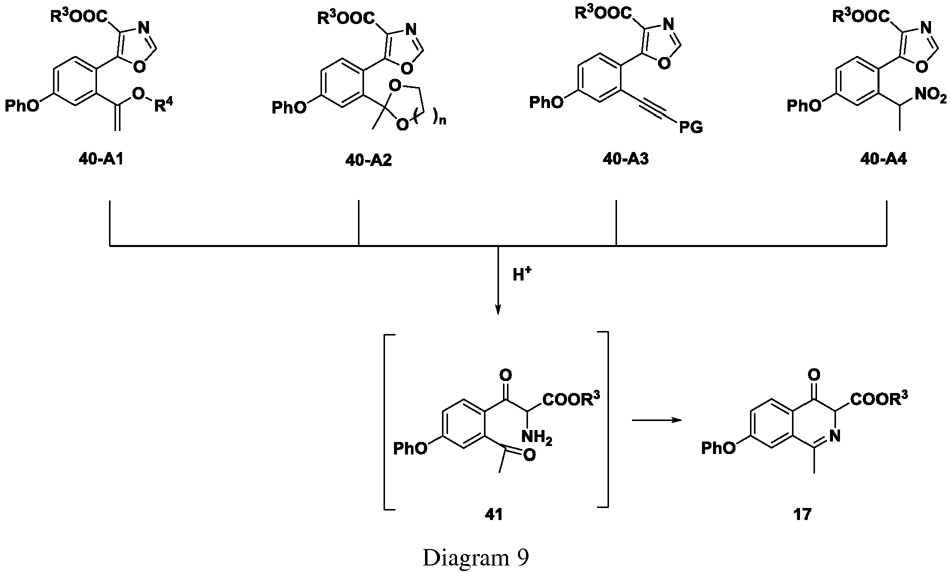

The obtained isoquinoline derivative of formula 17 is then converted to the roxadustat of formula 1 by the reaction with glycine in acetonitrile at 50°C by the effect of a base, which is DBU in this case (1,8-diazabicyclo[5.4.0]undec-7-ene) (Diagram 10). This reaction has a benefit over the present arrangements, both in its simplicity, in a lower temperature, and further in that a pressure vessel is not required to carry it out.

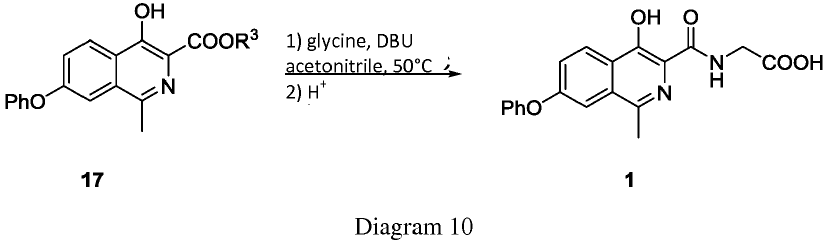

The subject matter of the invention will be further clarified by the following examples, which, however, have no effect on the scope of the invention as defined in the claims and are purely illustrative and do not limit the scope of the invention in any way.

### Examples

### EXAMPLE 1

### 2-Bromo-4-phenoxybenzoic acid of formula 38a

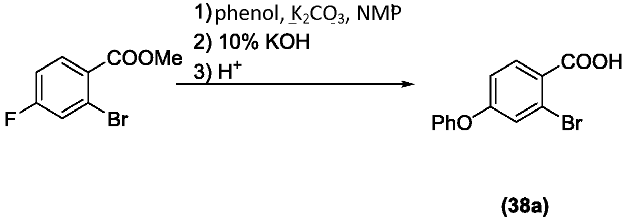

A mixture of methyl 2-bromo-4-fluorobenzoate (70 g, 0.3 mol), phenol (29.7 g, 0.32 mol) and potash (49.8 g, 0.36 mol) in *N*-methylpyrrolidone was heated at 95°C for the time of 8 h until complete conversion was complete. The reaction mixture was then cooled to 80°C, after the addition of 10% solution of KOH (250 ml), stirred for further 2 hours and then cooled to 25°C. Organic impurities were extracted into *tert*-butyl (methyl) ether (MTBE) (300 ml) and the aqueous layer was acidified with dilute HCl (conc. HCl / water; 1/4) until all product was precipitated in the form of white flakes. After suction, thorough washing with water and subsequent drying, the desired product was obtained in a form of white crystals in the yield of 88% and HPLC purity of 99%, m.p. 159.8-167.4°C.
¹H NMR (500 MHz, DMSO-d₆): δ = 7.05 (dd, J = 8.55 Hz, 2.35 Hz, 1H), 7.20 (d, J = 8.35 Hz, 2H), 7.28 (d, J = 2.49 Hz, 1H), 7.31 (t, J = 7.42 Hz, 1H), 7.52 (t, J = 7.70 Hz, 2H), 7.88 (d, J = 8.71 Hz, 1H), 13.26 (s, 1H),. ¹³C NMR (125 MHz, DMSO-d₆): δ = 116.2; 120.1; 121.9; 122.4; 125.1; 126.9; 130.4; 133.1; 154.6; 159.8; 166.3; HRMS (APCI, m/z): calculation for C₁₃H₉BrO₃ [M+H] 292.9913, found: 292.9916.

### EXAMPLE 2

### Ethyl 5-(2-bromo-4-phenoxyphenyl)oxazole-4-carboxylate of formula 39a

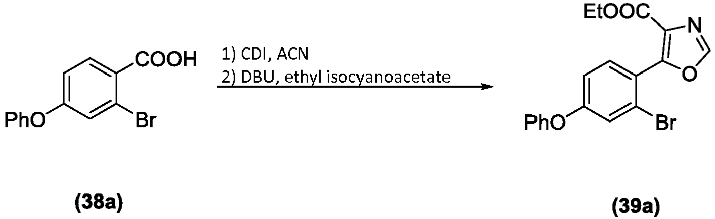

CDI (12.17 g, 0.075 mol) was added to a solution of 2-bromo-4-phenoxybenzoic acid (**38a**; 20 g; 0,068 mol) in acetonitrile (120 ml) and the mixture was stirred at room temperature for 3-4 h until all the acid was converted to the corresponding imidazole adduct (HPLC monitoring). Then, a solution of ethyl isocyanoacetate (8.2 ml, 0.075 mol) in ACN (20 ml) and DBU solution (11.22 ml, 0.075 mol) in acetonitrile (20 ml) was added and the reaction mixture was stirred for additional 6 hours at room temperature. The reaction mixture was concentrated to about 1/3 volume, water (100 ml) was added and an extraction by MTBE (2x80 ml) was performed. The organic portion was washed with 10% solution of Na₂CO₃ (80 ml), water (80 ml) and then anhydrous MgSO₄ was added as a drying agent. After the subsequent filtration, the solvent was distilled off and the crystallization of the residue from isopropanol (small amount, about 15-20 ml) gave 19.6 g of white crystals of the product in the yield of 74%, HPLC purity of 99%, m.p. 68.2-72.7°C.
¹H NMR (500 MHz, DMSO-d₆): δ = 4.2 (q, J = 7.03 Hz, 2H), 7.13 (dd, J = 8.42 Hz, 2.00 Hz, 1H), 7.20 (d, J = 7.94 Hz, 2H), 7.31 (t, J = 7.00 Hz, 1H), 7.40 (d, J = 1.94 Hz, 1H), 7.52 (t, J = 7.95 Hz, 2H), 4.66 (d, J = 8.42 Hz, 1H), 8.68 (s, 1H). ¹³C NMR (125 MHz, DMSO-d₆): δ = 116.5; 119.9; 121.37; 122.9; 123.9; 125.0; 128.6; 130.4; 134.0; 151.9; 153.1; 154.9; 159.4; 160.5; HRMS (APCI, m/z): calculation for C₁₈H₁₅BrNO₄ [M+H] 388.0184, found: 388.0178.

### EXAMPLE 3

### Ethyl 5-(2-(1-butoxyvinyl)-4-phenoxyphenyl)oxazole-4-carboxylate of formula 40-A1a

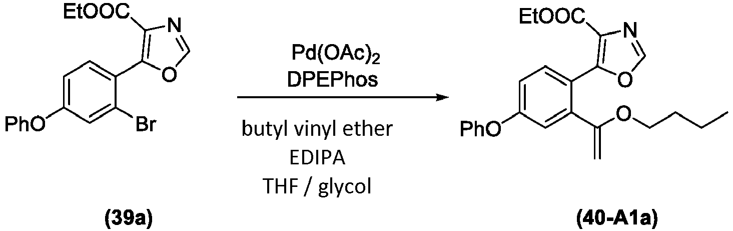

To a solution of the oxazole derivative (**39a**; 7.76 g; 20 mmol), EDIPA (*N,N-*Diisopropylethylamine) (11.1 ml; 64.8 mmol), butyl vinyl ether (10.01 mL, 78.2 mmol) in a mixture of THF / ethylene glycol (1:1; 100 ml) palladium acetate (179 mg, 0.8 mmol) and DPEPhos (bis [(2-diphenylphosphino) phenyl] ether) (516 mg, 0.96 mmol) was added and the resulting reaction mixture was stirred at 90°C for 8 h. Then, water (100 ml), ethyl acetate (EtOAc, 1x100 ml) were added to the cooled reaction mixture, and filtration was performed before the extraction by EtOAc (2 x 100 ml). The combined organic portions were washed with a 10% solution of Na₂CO₃ (100 ml) and water (100 ml). An activated charcoal was added, with which the organic portion was briefly boiled, filtration was carried out and the solvent was distilled off. The evaporation residue in form of orange oil weighed 10.57 g (HPLC purity of 55%) and was used for the next reaction step without further purification.

### EXAMPLE 4

### Ethyl 5-(2-(1-ethoxyvinyl)-4-phenoxyphenyl)oxazole-4-carboxylate of formula 40-A1b

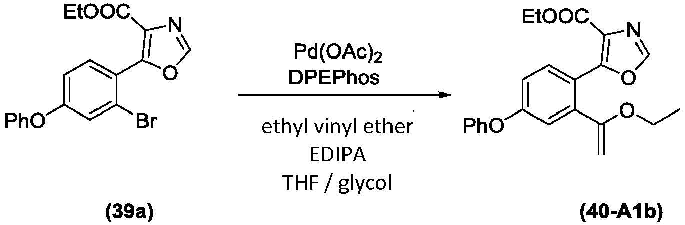

By the procedure described in Example 3 using the ethyl vinyl ether, the intermediate **40-A1b** was obtained in the form of 6.5 g of the mixture (HPLC purity of 58%), which was used without further purification for the subsequent reaction .

### EXAMPLE 5

### Ethyl 5-(2-(2-methyl-1,3-dioxolan-2-yl)-4-phenoxyphenyl)oxazole-4-carboxylate of formula 40-A2a

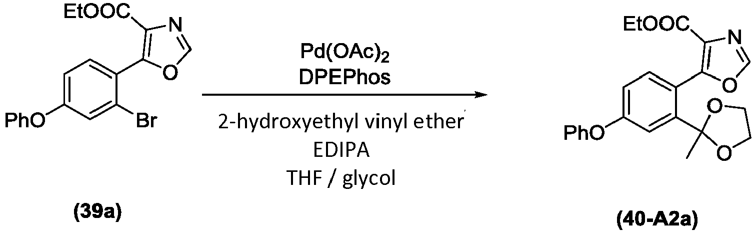

Palladium acetate (18 mg; 0.08 mmol) and DPEPhos (52 mg, 0.096 mmol) were added to a solution of the oxazole derivative (**39a**; 776 g; 2 mmol), EDIPA (1.11 ml; 6.48 mmol), 2-hydroxyethyl vinyl ether (0.70 ml; 7.82 mmol) in a mixture of THF / ethylene glycol (1:1; 20 ml) and the resulting reaction mixture was stirred at 90°C for 8 h. Water (20 ml), EtOAc (1x20 ml) were added to the cooled reaction mixture and filtration was performed before the extraction by EtOAc (2 x 20 ml). The combined organic portions were washed with a 10% solution of Na₂CO₃ (20 ml) and water (20 ml). An activated charcoal was added, with which the organic portion was briefly boiled, filtration was carried out and the solvent was distilled off. By the column chromatography (hexane / EtOAc, 6/4), it was obtained 230 mg (29%) of the product in a form of yellow oil, which was used immediately in the next reaction.

### EXAMPLE 6

### Ethyl 5-(2-(2-methyl-1,3-dioxepan-2-yl)-4-phenoxyphenyl)oxazole-4-carboxylate of formula 40-A2b

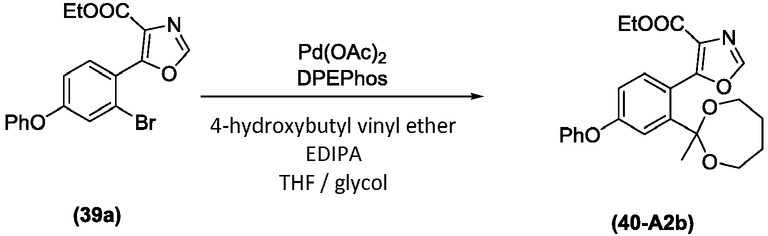

By the procedure described in Example 5 using the 4-hydroxybutyl vinyl ether, the intermediate **40-A2b** in the yield of 35% was obtained and it was immediately used in the following reaction.

### EXAMPLE 7

### Ethyl 5-(4-phenoxy-2-((trimethylsilyl) ethynyl) phenyl)oxazole-4-carboxylate of formula 40-A3a

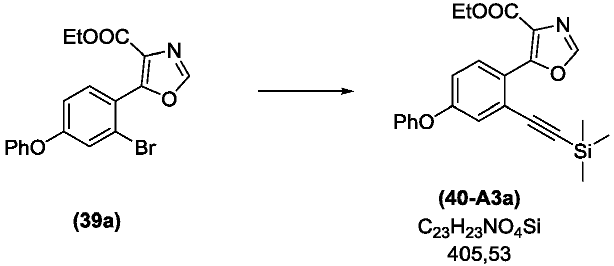

A flask containing the oxazole derivative (**39a**; 5 g; 12.88 mmol), CuI (200 mg, 1 mmol), and Pd(Ph₃P)₂Cl₂ (350 mg; 0.5 mmol) was evacuated, then, using a syringe toluene (25 ml), triethylamine (5 ml), and diisopropylamine (5 ml) were added gradually under the argon. After the repeated evacuation, trimethylsilylacetylene (5 ml, 36 mmol) was added dropwise under the argon and the mixture was stirred under the argon for 24 hours at the temperature of 80°C. The mixture was spilled over the pad of silica gel and after washing with ethyl acetate (25 ml) the filtrate was gradually washed with saturated solution of NH₄Cl (2 x 10 ml) and water (2 x 10 ml), dried with MgSO₄ with addition of activated charcoal and evaporated. The amount of 5.1 g of the yellowish evaporation product was obtained (HPLC purity of 96.9%, content of **39a** 2.2%), which was used for the next reaction stage without further purification.
¹H NMR (500 MHz, CDCl₃): δ = 0.12 (s, 9H), 1.28 (t, J = 7.1 Hz, 3H), 4.29 (q, J = 7.0 Hz, 2H), 7.03 (m, 3H), 7.41 (m, H), 7.20 (m, 2H), 7.51 (d, J = 8.4 Hz, 2H), 7.95 (s, 1H). HRMS (APCI, m/z): calculation for C₂₃H₂₄NO₄Si [M+H] 406.1475, found: 406.1462.

### EXAMPLE 8

### Ethyl-5-(4-phenoxy-2-((triisopropylsilyl) ethynyl) phenyl)oxazole-4-carboxylate with formula 40-A3a

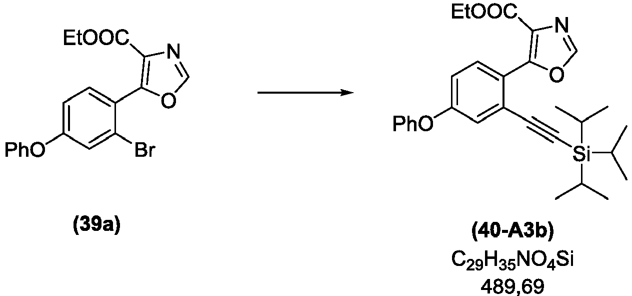

By the procedure described in Example 7 the intermediate **40-A3b** was obtained in a yield of 97% using the triisopropyl acetylene, which was used in a form of evaporation residue (HPLC purity of 95,3%) without further purification for the subsequent reaction.
HRMS (APCI, m/z): calculation for C₂₉H₃₆NO₄Si [M+H] 490.2414, found: 490.2427.

### EXAMPLE 9

### Ethyl 4-hydroxy-1-methyl-7-phenoxy-isoquinoline-3-carboxylate of formula 17b

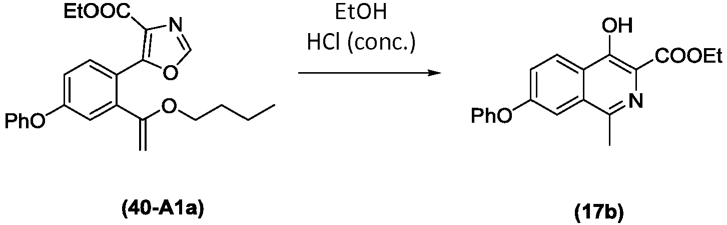

A solution of HCl in EtOH (10 ml of conc. HCl; 20 ml of EtOH) was added to a solution of the oxazole derivative (**40-A1a;** 10.57 g, HPLC purity of 55%) from the previous reaction in ethanol (EtOH, 60 ml) and the reaction mixture was heated for 6 hours at 60°C. The precipitated impurity from the previous reaction was sucked out of the reaction mixture, water (100 ml) was added, EtOH was evaporated and the extraction into EtOAc (2 x 100 ml) was performed. The combined organic portions were washed with salt brine (100 ml) and activated charcoal and drying agent were added, with which the portions were briefly boiled. After the filtration of the solid portions, the solvent was evaporated and by crystallization from isopropanol 3 g (46%) of the product was obtained in the form of finely yellowish crystals with the HPLC purity of 99%, m.p. 104.2-108.7°C.
¹H NMR (500 MHz, CDCl₃): δ = 1.51 (t, J = 7.10 Hz, 3H), 2.75 (s, 3H), 4.56 (q, J = 7.37 Hz, 2H), 7.12 (d, J = 7.88 Hz, 2H), 7.23 (t, J = 7.43 Hz, 1H), 7.38-7.54 (m, 4H), 8.35-8.45 (m, 1H), 11.88 (s, 1H). ¹³C NMR (125 MHz, CDCl₃): δ = 14.4; 22.3; 62.3; 111.6; 118.8; 120.0; 122.4; 123.7; 124.7; 126.0; 130.3; 132.4; 148.5; 155.8; 155.9; 159.3; 170.9; HRMS (APCI, m/z): calculation for C₁₉H₁₈NO₄ [M+H] 324.1236, found: 324.1233.

### EXAMPLE 10

### Ethyl 4-hydroxy-1-methyl-7-phenoxy-isoquinoline-3-carboxylate of formula 17b

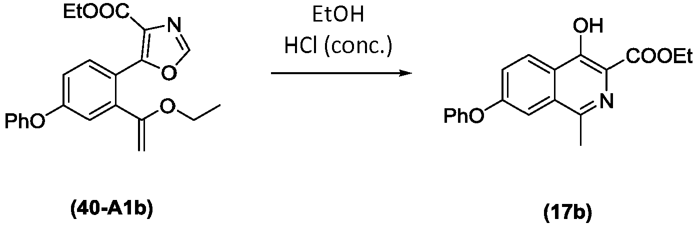

By the procedure described in Example 9 using the intermediate **40-A1b** a substance **17b** was obtained in a yield of 51% with HPLC purity of 99%.

### EXAMPLE 11

### Ethyl 4-hydroxy-1-methyl-7-phenoxy-isoquinoline-3-carboxylate of formula 17b

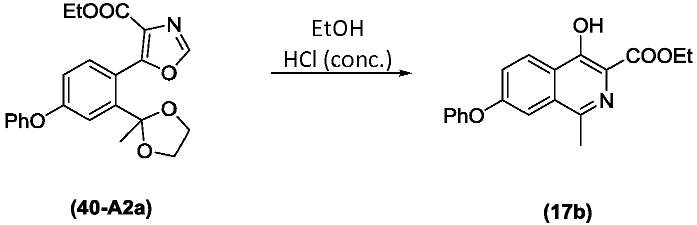

By the procedure described in Example 9 using the intermediate **40-A2a** a substance **17b** was obtained in a yield of 21% with HPLC purity of 99%.

### EXAMPLE 12

### Ethyl 4-hydroxy-1-methyl-7-phenoxy-isoquinoline-3-carboxylate of formula 17b

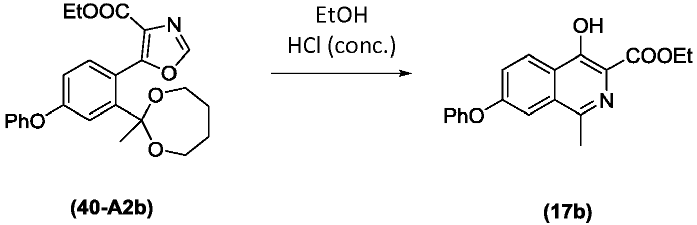

By the procedure described in Example 9 using the intermediate **40-A2a** a substance **17b** was obtained in a yield of 15% with HPLC purity of 99%.

### EXAMPLE 13

### Ethyl 5-(2-ethynyl-4-phenoxyphenyl)oxazole-4-carboxylate of formula 40-42a

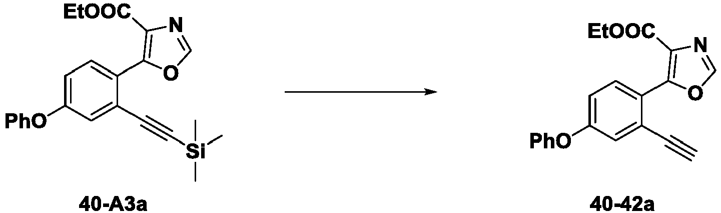

Starting substance **40-A3a** obtained by the procedure described in Example 7 (1.0 g) was dissolved in EtOH (10 ml) and after addition of dried KF (0.05 g) the mixture was stirred for 4 h at the laboratory temperature. The filtrate was completely evaporated to dryness, dissolved in EtOAc (10 ml), washed with salt brine (2 x 5 ml) and dried with MgSO₄. 0.8 g of a colorless evaporation residue (HPLC purity of 96.3%) was obtained, which was used without further purification for the subsequent reaction.
¹H NMR (500 MHz, CDCl₃): δ = 1.25 (t, J = 7.1 Hz, 3H), 3.01 (s, 1H), 4.25 (q, J = 7.0 Hz, 2H), 7.02 (m, 3H), 7.11 (m, 2H), 7.33 (m, H), 7.45 (d, J = 8.4 Hz, 2H), 8.68 (s, 1H). HRMS (APCI, m/z): calculation for C₂₀H₁₆NO₄ [M+H] 334.1079, found: 334.1089.

### EXAMPLE 14

### Ethyl 4-hydroxy-1-methyl-7-phenoxy-isoquinoline-3-carboxylate of formula 17b

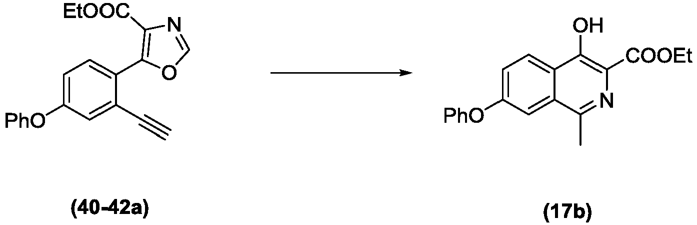

The starting compound **40-42a** (0.6 g, 1.2 mmol) obtained by the procedure described in Example 13 was divided into 6 vials and each of them dissolved in 1 ml of the appropriate solvent(C₂H₅OH or CF₃CH₂OH). After the addition of 0.1 ml of 35% of the appropriate aqueous acid (commercially available HCl was used, CF₃SO₂OH was diluted just before the use), the mixture was stirred at the temperature of 50°C for 48 hours. The reaction was monitored by TLC (pre-coated silica plates Merck, hexane-acetone 7:3) and HPLC. The results are summarized in Table I.

**Table I - Hydration and subsequent cyclization of intermediate 40-42a**

| **Des.** | **Solvent** | **Acid** | **HPLC contents** | |
|---|---|---|---|---|
| | | | **40-42A** | **17b** |
| A | C₂H₅OH | HCl | 22% | 6% |
| B | C₂H₅OH | CF₃SO₂OH | 18% | 18% |
| C | C₂H₅OH | HCl-CF₃SO₂OH 1:1 | 14% | 22% |
| D | CF₃CH₂OH | HCl | 27% | 29% |
| E | CF₃CH₂OH | CF₃SO₂OH | 6% | 59% |
| F | CF₃CH₂OH | HCl-CF₃SO₂OH 1:1 | 2% | 77% |

### EXAMPLE 15

### Ethyl 4-hydroxy-1-methyl-7-phenoxy-isoquinoline-3-carboxylate of formula 17b

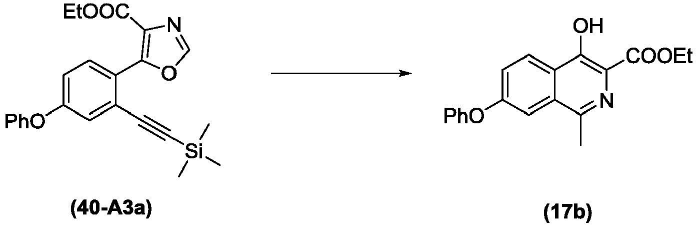

Starting substance **40-A3a** obtained by the procedure described in Example 7 (4.0 g, 9.9 mmol) was dissolved in CF₃CH₂OH (10 ml) and after addition of 1M solution of Bu₄N⁺F⁻ (0.5 ml) the mixture was mixed for 2 hours at the laboratory temperature. Then, a mixture of concentrated HCl (0.5 ml) and CF₃SO₂OH (0.5 ml) was added and the mixture was stirred under nitrogen atmosphere with low reflux for 48 hours. After cooling down, the mixture was diluted by EtOAc (25 ml), washed by salt brine (2 x 5 ml) and dried by MgSO₄. 3.1 g of evaporation residue was obtained, containing 76.9% of product, in accordance with HPLC. Using a flash chromatography (silica gel Merck 60; hexane-acetone 9:1) an amount of 1.8 g of the substance **17b** (56%) was obtained with HPLC purity of 96,8%.

### EXAMPLE 16

### Ethyl 4-hydroxy-1-methyl-7-phenoxy-isoquinoline-3-carboxylate of formula 17b

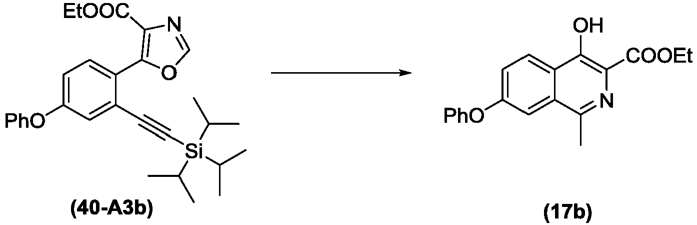

By the procedure described in Example 15 using the intermediate **40-A3b** a substance **17b** was obtained in a yield of 52% with HPLC purity of 97,1%.

### EXAMPLE 17

### 7-Phenoxy-4-hydroxy-1-methyl-isoquinoline-3-carbonyl)glycine of formula 1

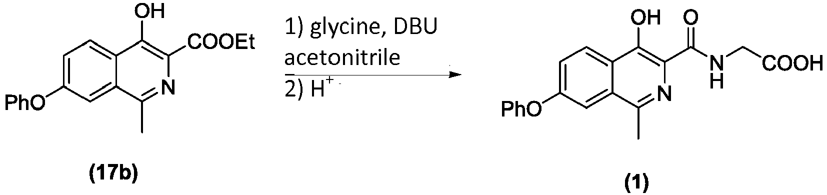

A mixture of ester (**17b**; 161 mg, 0.5 mmol), glycine (83 mg, 1.1 mmol), acetonitrile (4 ml) and DBU (0.16 ml; 1.1 mmol) was heated to 60°C for 6 h (the reaction mixture darkened significantly). Water (10 ml), EtOAc (10 ml) and dilute HCl (conc. HCl / water; 1/5; 5 ml) were then added to the cooled reaction mixture. The aqueous phase was poured off and the organic phase was washed with water (10 mL) and 10% solution of Na₂CO₃ (5 ml). The new aqueous phase was gradually acidified with dilute HCl (conc. HCl / water; 1/5) until crystallization was initiated (slightly acidic pH), and after 30 minutes of stirring, the crystals of the body color (HPLC 100%), 140 mg (79%) were sucked out, m.p. 208.7-213.5°C.
¹H NMR (500 MHz, DMSO-d₆): δ = 2.74 (s, 3H), 4.09 (d, J = 6.24 Hz, 2H), 7.22 (d, J = 8.71 Hz, 2H), 7.29 (t, J = 7.67 Hz, 1H), 7.52 (t, J = 8.58 Hz, 2H), 7.56 (t, J = 7.56, 2,73 Hz, 1H), 7.65 (d, J = 7.64 Hz, 1H), 8.32 (d, J = 9.20 Hz, 1H), 9.14 (t, J = 6.34 Hz, 1H), 12.84 (s, 1H), 13.33 (s, 1H). ¹³C NMR (125 MHz, DMSO-d6): δ = 21.5; 112.1; 119.4; 119.5; 122.4; 123.5; 124.5; 125.2; 130.4; 131.4; 146.9; 152.8; 155.5; 157.8; 169.9; 170.8; HRMS (APCI, m/z): calculation for C19H17N2O5 [M+H] 353.1137, found: 353.1132.

## Claims

1. A method for preparing the intermediate of roxadustat of formula **34,** 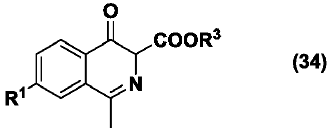 ***characterized in that*** the intermediate of formula **32** 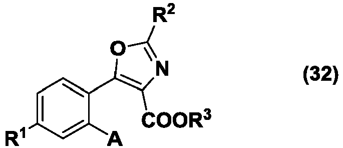 is converted in an acidic medium to an intermediate of formula **33,** which spontaneously cyclizes to the intermediate of formula **34,** 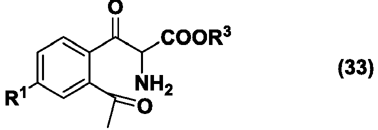 wherein
R¹ is halogen, PhO, OH or a protected hydroxyl group;
R² is H or a C1 -C10 alkyl group;
R³ is H or a C1 -C10 alkyl group or aryl group;
A is a precursor of an acetyl group, preferably a group
A1, wherein R⁴ is a C1-C10 alkyl group;
A2, where n = 1 to 5;
A3, wherein PG is a protecting group, preferably SiR⁵R⁶R⁷ or CR⁸R⁹OH, where R⁵ to R⁹ is a C1-C10 alkyl group;
or A4

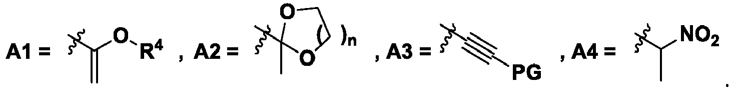

2. A method of preparing according to claim 1 **characterized in that** the intermediate of formula **32** 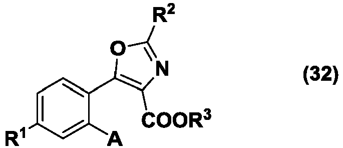 is prepared by the coupling reaction of intermediate 31 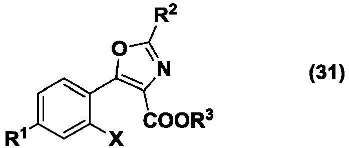 with a compound selected from the group consisting of alkyl vinyl ethers, hydroxyalkyl vinyl ethers, nitroethane, trialkylsilyl acetylenes and 2,2-dialkyl derivatives of prop-2-yn-1-ol, wherein
X is I, Br or Cl, eventually other groups suitable for coupling reactions such as OTf;
R¹ is halogen, PhO, OH or a protected hydroxyl group;
R² is H or a C1 -C10 alkyl group;
R³ is H or a C1 -C10 alkyl group or aryl group;
A is a precursor of an acetyl group, preferably a group
A1, wherein R⁴ is a C1-C10 alkyl group;
A2, where n = 1 to 5;
A3, wherein PG is a protecting group, preferably SiR⁵R⁶R⁷ or CR⁸R⁹OH, where R⁵ to R⁹ is a C1-C10 alkyl group;
or A4

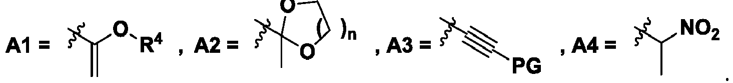

3. A method of preparing according to claim 2, ***characterized in that*** the intermediate of formula **31** is a compound of formula **39**

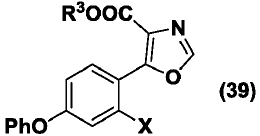

4. A method of preparing according to claim 3 ***characterized in that*** the intermediate of formula **39** 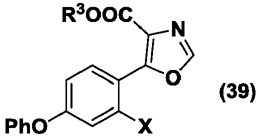 is prepared by a process comprising the steps
a) reaction of the compound of formula **37** 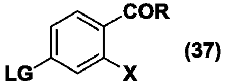 with phenol followed by hydrolysis to give the compound of formula **38,** 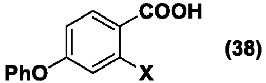
b) reaction of compound with formula **38** with cyanoacetate of formula 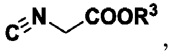 after the activation of the carboxyl group with a suitable activating agent, preferably 1,1'-carbonyldiimidazole, to give the intermediate of formula **39,** wherein
X is I, Br or Cl, eventually other groups suitable for coupling reactions such as OTf;
R is alkoxy group, aryloxy group, NH₂, N(alkyl)₂, N(aryl)₂, N(H)(alkyl), or N(H)(aryl);
R³ is H, C1-C10 alkyl group or aryl group;
LG is -NO₂, N₂⁺, I, Br, Cl, F, B(OH)₂, B(OR)₂, eventually other groups suitable for coupling reactions such as OTf;

5. A method for preparing according to claim 4 ***characterized in that*** the intermediate of formula **39a** 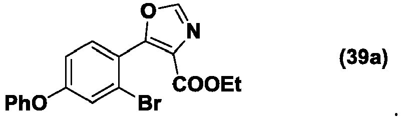 is prepared by a process comprising the steps
a) reaction of methyl 2-bromo-4-fluorobenzoate of formula 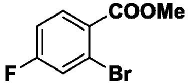 with phenol followed by hydrolysis to give 2-bromo-4-phenoxybenzoic acid of formula **38a** 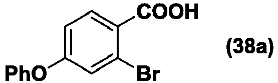
b) reaction of a compound of formula **38a** with ethyl isocyanoacetate upon activation of the carboxyl group with a suitable activating agent, preferably 1,1'-carbonyldiimidazole, to form the intermediate of formula **39a.**

6. A method of preparing according to claim 1 and 2 ***characterized in that*** the intermediate of formula **32** is a compound of formula **40-A1,** 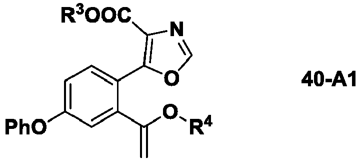 which, in an acidic environment, is subject to the opening of the oxazole ring, hydrolysis of the vinyl ether group to the acetyl group and subsequent condensation to give the intermediate **17,** 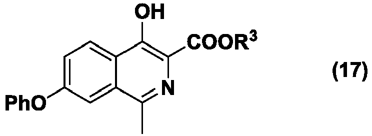 wherein R³ and R⁴ is a C1-C10 alkyl group.

7. A method of preparing according to claim 1 and 2 ***characterized in that*** the intermediate of formula **32** is a compound of formula **40-A2,** 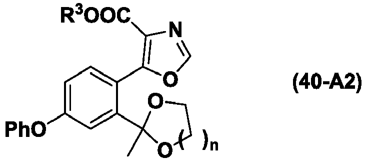 which, in an acidic medium, is subject to the opening of the oxazole ring, hydrolysis of the dioxolane group to the acetyl group and subsequent condensation to give the intermediate **17,** 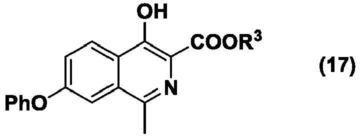 wherein R³ is a C1-C10 alkyl group and n = 1 to 5.

8. A method of preparing according to claim 1 and 2 ***characterized in that*** the intermediate of formula **32** is a compound of formula **40-A3,** 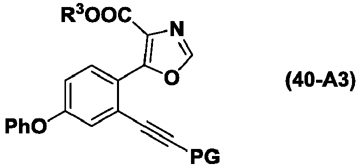 which, in an acidic environment, is subject to the opening of the oxazole ring, deprotection of the acetylenic grouping, its hydrolysis to the acetyl group and subsequent condensation to give the intermediate **17,** 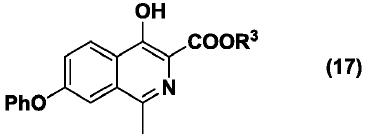 wherein R³ is C1-C10 alkyl group and PG is suitable protective group, preferably R⁵R⁶R⁷Si, wherein alkyl groups R⁵, R⁶ and R⁷ are C1-C10 alkyl groups.

9. A method of preparing according to claim 1 and 2 ***characterized in that*** the intermediate of formula **32** is a compound of formula **40-A4,** 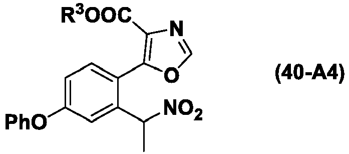 which, in an acidic environment, is subject to the opening of the oxazole ring, hydrolysis of the nitroalkyl grouping to the acetyl group and subsequent condensation to give the intermediate **17,** 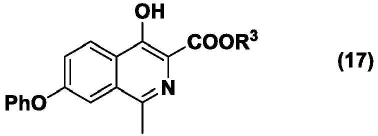 wherein R³ is a C1-C10 alkyl group.

10. A method of preparing according to claims 6 to 9, ***characterized in that,*** the intermediate of formula **17**, 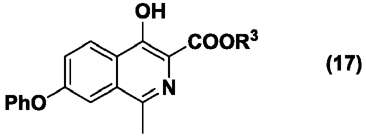 is converted by reaction with glycine or its functional derivatives to roxadustat of formula **1**

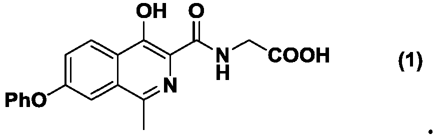

11. Ethyl 5-(2-bromo-4-phenoxyphenyl)oxazole-4-carboxylate of formula **39a.**

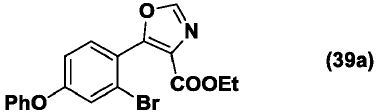

12. Ethyl 3-(2-acetyl-4-phenoxyphenyl)-2-amino-3-oxopropanoate of formula **33.**

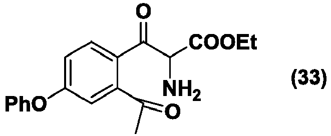

13. Ethyl 5-(2-(1-butoxyvinyl)-4-phenoxyphenyl)oxazole-4-carboxylate of formula **40-A1a.**

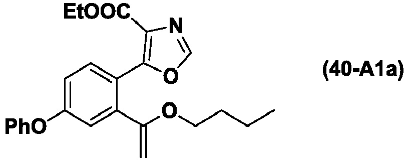

14. Ethyl 5-(2-(1-ethoxyvinyl)-4-phenoxyphenyl)oxazole-4-carboxylate of formula **40-A1b.**

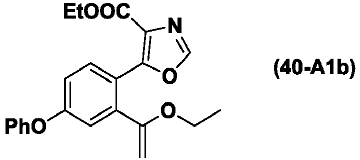

15. Ethyl5-(2-(2-methyl-1,3-dioxolan-2-yl)-4-phenoxyphenyl)oxazole-4-carboxylate of formula **40-A2a.**

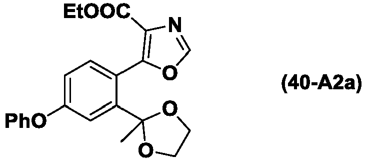

16. Ethyl 5-(2-(2-methyl-1,3-dioxepan-2-yl)-4-phenoxyphenyl)oxazole-4-carboxylate of formula **40-A2b.**

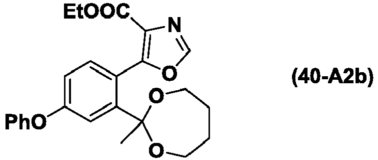

17. Ethyl 5-(4-phenoxy-2-((trimethylsilyl)ethynyl)phenyl)oxazole-4-carboxylate of formula **40-A3a.**

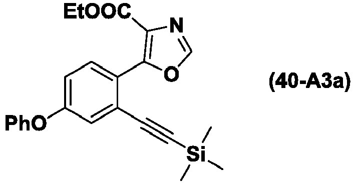

18. Ethyl 5-(4-phenoxy-2-((triisopropylsilyl)ethynyl)phenyl)oxazole-4-carboxylate of formula **40-A3b.**

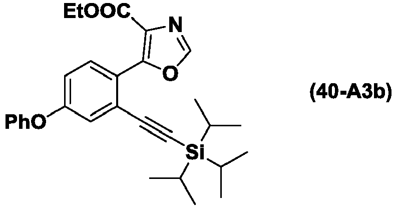

19. Ethyl 5-(2-ethynyl-4-phenoxyphenyl)oxazole-4-carboxylate of formula **40-42a**

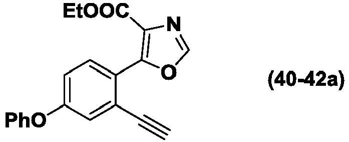

## Patentansprüche

1. Ein Verfahren zur Herstellung des Zwischenproduktes von Roxadustat der Formel **34,** 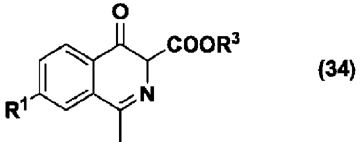 ***dadurch gekennzeichnet, dass*** das Zwischenprodukt der Formel **32** 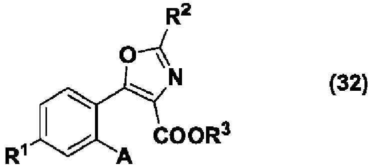 in saurem Medium zu einem Zwischenprodukt der Formel **33** umgewandelt wird, das spontan zum Zwischenprodukt der Formel **34** cyclisiert, 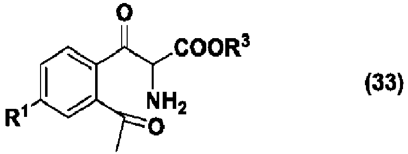 worin
R¹ Halogen, PhO, OH oder eine geschützte Hydroxylgruppe ist;
R² H oder eine C1-C10-Alkylgruppe ist;
R³ H oder eine C1-C10-Alkylgruppe oder -Arylgruppe ist;
A ein Vorläufer einer Acetylgruppe ist, vorzugsweise eine Gruppe
A1, worin R⁴ eine C1 -C10-Alkylgruppe ist;
A2, worin n = 1 bis 5 ist;
A3, worin PG eine Schutzgruppe ist, vorzugsweise SiR⁵R⁶R⁷ oder CR⁸R⁹OH, wobei R⁵ bis R⁹ eine C1-C10-Alkylgruppe ist;
oder A4

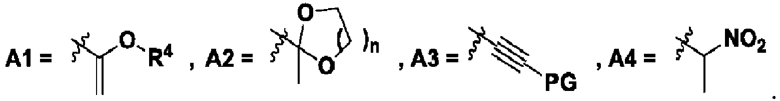

2. Verfahren zur Herstellung nach Anspruch 1, **dadurch gekennzeichnet, dass** das Zwischenprodukt der Formel **32** 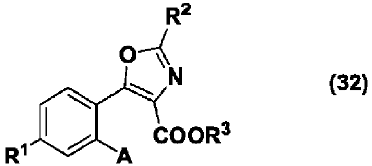 durch die Kupplungsreaktion des Zwischenprodukts 31 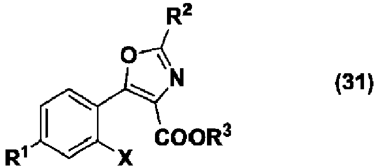 mit einer Verbindung, ausgewählt aus der Gruppe bestehend aus Alkylvinylethern, Hydroxyalkylvinylethern, Nitroethan, Trialkylsilylacetylenen und 2,2-Dialkylderivaten von Prop-2-in-1-ol, hergestellt wird, wobei
X I, Br oder Cl ist, gegebenenfalls andere Gruppen, die für Kupplungsreaktionen geeignet sind, wie z. B. OTf;
R¹ Halogen, PhO, OH oder eine geschützte Hydroxylgruppe ist;
R² H oder eine C1-C10-Alkylgruppe ist;
R³ H oder eine C1-C10-Alkylgruppe oder -Arylgruppe ist;
A ein Vorläufer einer Acetylgruppe ist, vorzugsweise eine Gruppe
A1, worin R⁴ eine C1-C10-Alkylgruppe ist;
A2, worin n = 1 bis 5 ist;
A3, worin PG eine Schutzgruppe ist, vorzugsweise SiR⁵R⁶R⁷ oder CR⁸R⁹OH, wobei R⁵ bis R⁹ eine C1-C10-Alkylgruppe ist;
oder A4

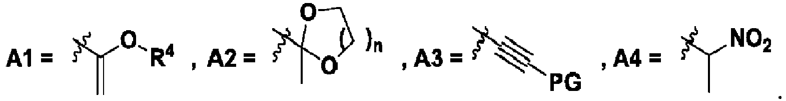

3. Verfahren zur Herstellung nach Anspruch 2, ***dadurch gekennzeichnet, dass*** das Zwischenprodukt der Formel **31** eine Verbindung der Formel **39** ist

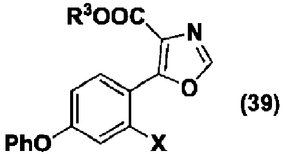

4. Verfahren zur Herstellung nach Anspruch 3, ***dadurch gekennzeichnet, dass*** das Zwischenprodukt der Formel **39** 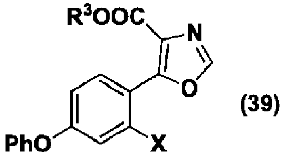 durch ein Verfahren hergestellt wird, das die Schritte umfasst:
a) Umsetzung der Verbindung der Formel **37** 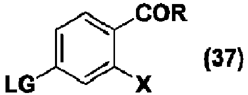 mit Phenol, gefolgt von Hydrolyse zur Verbindung der Formel **38** , 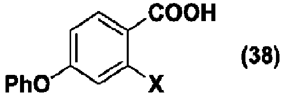
b) Umsetzung der Verbindung der Formel **38** mit Cyanoacetat der Formel 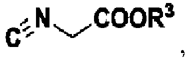 nach der Aktivierung der Carboxylgruppe mit einem geeigneten Aktivierungsmittel, vorzugsweise 1,1'-Carbonyldiimidazol, zu dem Zwischenprodukt der Formel **39**, worin
X I, Br oder Cl, gegebenenfalls andere Gruppen, die für Kupplungsreaktionen geeignet sind, wie z. B. OTf, ist;
R eine Alkoxygruppe, Aryloxygruppe, NH₂, N(Alkyl)₂, N(Aryl)₂, N(H)(Alkyl) oder N(H)(Aryl) ist;
R³ H, eine C1-C10-Alkylgruppe oder -Arylgruppe ist;
LG -NO₂, N₂⁺, I, Br, Cl, F, B(OH)₂, B(OR)₂, gegebenenfalls andere, für Kupplungsreaktionen geeignete Gruppen, wie OTf, ist.

5. Verfahren zur Herstellung nach Anspruch 4, ***dadurch gekennzeichnet, dass*** das Zwischenprodukt der Formel **39a** 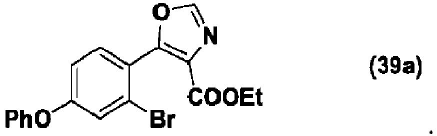 durch ein Verfahren hergestellt wird, das die Schritte umfasst:
a) Umsetzung von 2-Brom-4-fluorbenzoesäuremethylester der Formel 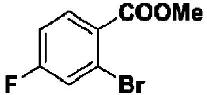 mit Phenol, gefolgt von Hydrolyse zu 2-Brom-4-phenoxybenzoesäure der Formel **38a** zu ergeben 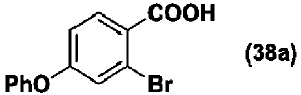
b) Umsetzung einer Verbindung der Formel **38a** mit Ethylisocyanoacetat nach Aktivierung der Carboxylgruppe mit einem geeigneten Aktivierungsmittel, vorzugsweise 1,1'-Carbonyldiimidazol, zum Zwischenprodukt der Formel **39a.**

6. Verfahren zur Herstellung nach Anspruch 1 und 2, ***dadurch gekennzeichnet, dass*** das Zwischenprodukt der Formel **32** eine Verbindung der Formel **40-A1** ist, 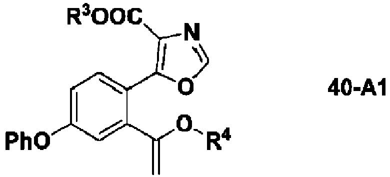 welche in saurer Umgebung der Öffnung des Oxazolrings, der Hydrolyse der Vinylethergruppe zur Acetylgruppe und der anschließenden Kondensation zum Zwischenprodukt **17** unterworfen wird, 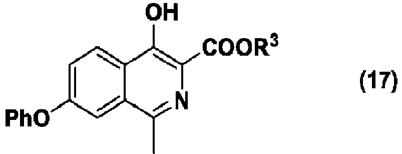 worin R³ und R⁴ eine C1-C10-Alkylgruppe sind.

7. Verfahren zur Herstellung nach Anspruch 1 und 2, ***dadurch gekennzeichnet, dass*** das Zwischenprodukt der Formel **32** eine Verbindung der Formel **40-A2** ist, 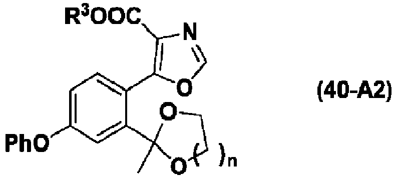 die in einem sauren Medium der Öffnung des Oxazolrings, der Hydrolyse der Dioxolangruppe zur Acetylgruppe und der anschließenden Kondensation zum Zwischenprodukt **17** unterworfen wird, 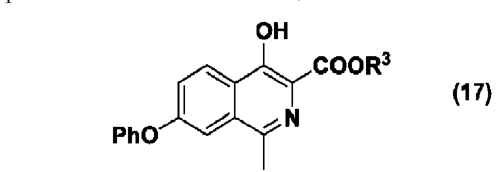 worin R³ eine C1-C10-Alkylgruppe ist und n = 1 bis 5 ist.

8. Verfahren zur Herstellung nach Anspruch 1 und 2, ***dadurch gekennzeichnet, dass*** das Zwischenprodukt der Formel **32** eine Verbindung der Formel **40-A3** ist, 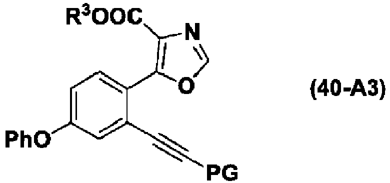 welche in saurer Umgebung der Öffnung des Oxazolrings, dem Entfernen der Schutzgruppe der Acetylengruppe, deren Hydrolyse zur Acetylgruppe und der anschließenden Kondensation zum Zwischenprodukt **17** unterworfen wird, 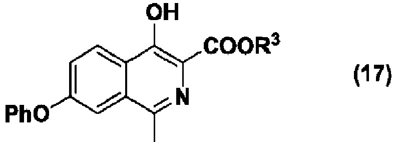 worin R³ eine C1-C10-Alkylgruppe ist und PG eine geeignete Schutzgruppe, vorzugsweise, R⁵,R⁶,R⁷Si ist, wobei die Alkylgruppen R⁵, R⁶ und R⁷ C1-C10-Alkylgruppen sind.

9. Verfahren zur Herstellung nach Anspruch 1 und 2, ***dadurch gekennzeichnet, dass*** das Zwischenprodukt der Formel **32** eine Verbindung der Formel **40-A4** ist, 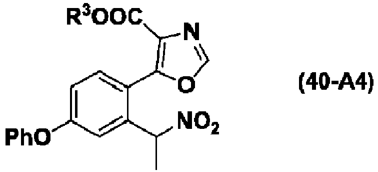 welche in saurer Umgebung der Öffnung des Oxazolrings, der Hydrolyse der Nitroalkylgruppe zur Acetylgruppe und der anschließenden Kondensation zum Zwischenprodukt **17** unterworfen wird, 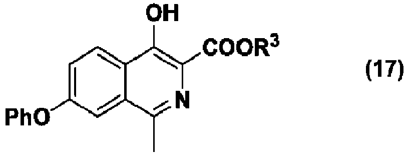 worin R³ eine C1-C10-Alkylgruppe ist.

10. Verfahren zur Herstellung nach den Ansprüchen 6 bis 9, ***dadurch gekennzeichnet,* dass** das Zwischenprodukt der Formel **17** 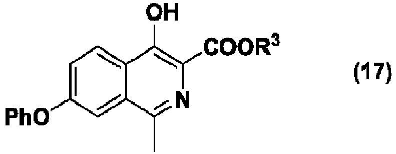 durch Umsetzung mit Glycin oder seinen funktionellen Derivaten zu Roxadustat der Formel **1** umgewandelt wird

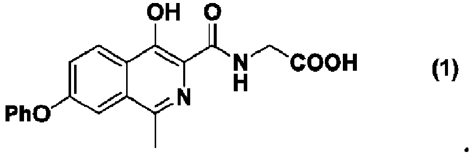

11. 5-(2-Brom-4-phenoxyphenyl)-oxazol-4-carbonsäureethylester der Formel **39a.**

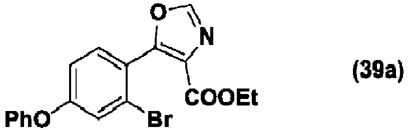

12. 3-(2-Acetyl-4-phenoxyphenyl)-2-amino-3-oxopropansäureethylester der Formel **33.**

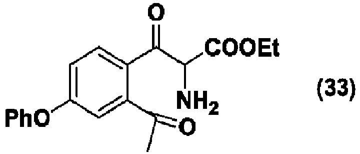

13. 5-(2-(1-Butoxyvinyl)-4-phenoxyphenyl)oxazol-4-carbonsäureethylester der Formel **40-A1a.**

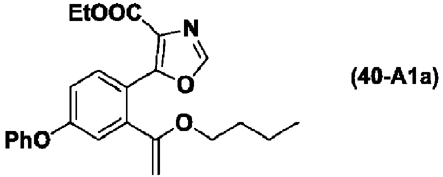

14. 5-(2-(1-Ethoxyvinyl)-4-phenoxyphenyl)-oxazol-4-carbonsäureethylester der Formel **40-A1b.**

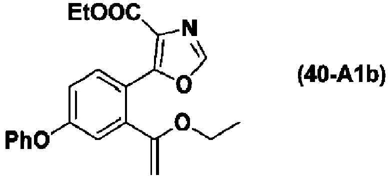

15. 5-(2-(2-Methyl-1,3-dioxolan-2-yl)-4-phenoxyphenyl)oxazol-4-carbonsäureethylester der Formel **40-A2a.**

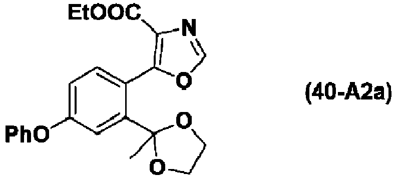

16. 5-(2-(2-Methyl-1,3-dioxepan-2-yl)-4-phenoxyphenyl)oxazol-4-carbonsäureethylester der Formel **40-A2b.**

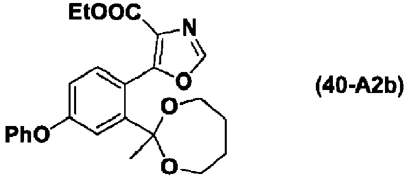

17. 5-(4-Phenoxy-2-((trimethylsilyl)ethinyl)phenyl)oxazol-4-carbonsäureethylester der Formel **40-A3a.**

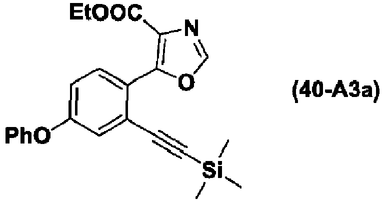

18. 5-(4-Phenoxy-2-((triisopropylsilyl)ethinyl)phenyl)oxazol-4-carbonsäureethylester der Formel **40-A3b.**

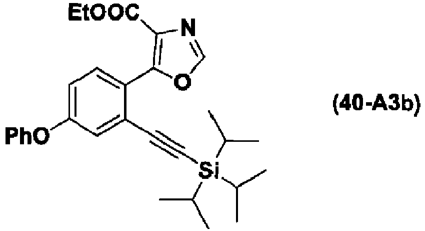

19. 5-(2-Ethinyl-4-phenoxyphenyl)-oxazol-4-carbonsäureethylester der Formel **40-42a.**

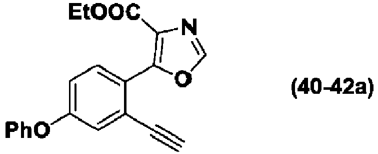

## Revendications

1. Un procédé de préparation de l'intermédiaire du roxadustat de formule 34, 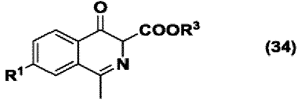 **caractérisé en ce que** l'intermédiaire de formule 32 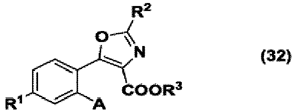 est converti dans un milieu acide en un intermédiaire de formule 33, qui spontanément se cyclise en l'intermédiaire de formule 34, 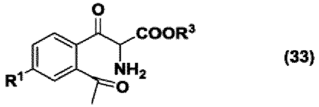 où
R¹ est un halogène, PhO, OH ou un groupe hydroxyle protégé;
R² est H ou un groupe alkyle en C₁-C₁₀;
R² est H ou un groupe alkyle en C₁-C₁₀ ou un groupe aryle;
A est un précurseur d'un groupe acétyle, de préférence un groupe
A1, dans lequel R⁴ est un groupe alkyle en C₁-C₁₀;
A2, où n = 1 à 5;
A3, où PG est un groupe protecteur, de préférence SiR⁵R⁶R⁷ ou CR⁸R⁹OH, où R⁵ à R⁹ sont des groupes alkyles en C₁-C₁₀; ou
A4

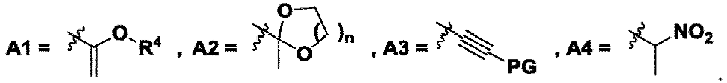

2. Un procédé de préparation selon la revendication 1, **caractérisé en ce que** l'intermédiaire de formule 32 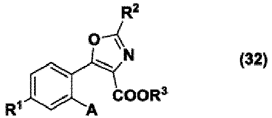 est préparé par la réaction de couplage de l'intermédiaire 31 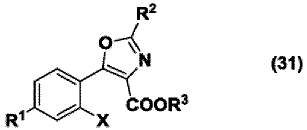 avec un composé choisi dans le groupe constitué par les éthers d'alkylvinyle, les éthers d'hydroxyalkyl vinyliques, le nitroéthane, les trialkylsilyl acétylènes et les dérivés 2,2-dialkyliques du prop-2-yn-1-ol, dans lesquels:
X est I, Br ou Cl, éventuellement d'autres groupes appropriés pour des réactions de couplage telles que OTf;
R² est un halogène, PhO, OH ou un groupe hydroxyle protégé;
R² est H ou un groupe alkyle en C₁-C₁₀;
R² est H ou un groupe alkyle en C₁-C₁₀ ou un groupe aryle;
A est un précurseur d'un groupe acétyle, de préférence un groupe
Al, dans lequel R⁴ est un groupe alkyle en C₁-C₁₀;
A2, où n = 1 à 5;
A3, où PO est un groupe protecteur, de préférence SiR⁵R⁶R⁷ ou CR⁸R⁹OH, où R⁵ à R⁹ sont des groupes alkyles en C₁-C₁₀; ou
A4

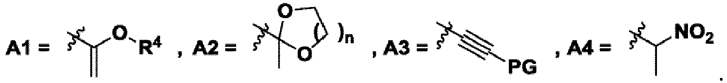

3. Un procédé de préparation selon la revendication 2, **caractérisé en ce que** l'intermédiaire de formule 31 est un composé de formule 39

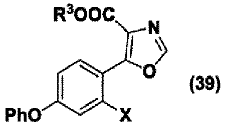

4. Un procédé de préparation selon la revendication 3, **caractérisé en ce que** l'intermédiaire de la formule 39 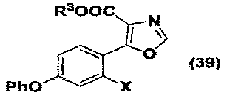 est préparé par un procédé comprenant les étapes de
a) réaction du composé de formule 37 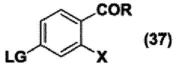 avec du phénol puis hydrolyse pour donner le composé de formule 38, 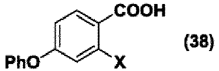
b) réaction du composé de formule 38 avec du cyanoacétate de formule 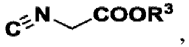 après l'activation du groupe carboxyle avec un agent d'activation approprié, de préférence le 1,1'-carbonyldiimidazole, pour donner l'intermédiare de formule 39, dans laquelle
- X est I, Br ou Cl, éventuellement d'autres groupes appropriés pour des réactions de couplage telles que OTf;
- R est un groupe alcoxy, un groupe aryloxy, NH₂, N(alkyl)₂, N(aryl)₂, N(H) (alkyle) ou N(H) (aryle);
- R³ est H, un groupe alkyle en C₁-C₁₀ ou un groupe aryle;
- LG est -NO₂, N₂⁺, I, Br, Cl, F, B(OH)₂, B(OR)₂, éventuellement d'autres groupes appropriés pour des réactions de couplage telles que OTf;

5. Un procédé de préparation selon la revendication 4 **caractérisé en ce que** l'intermédiaire de formule 39a 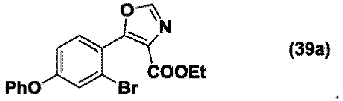 est préparé par un procédé comprenant les étapes de:
a) réaction du méthyl 2-bromo-4-fluorobenzoate de formule 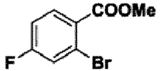 avec du phénol puis hydrolyse pour donner l'acide 2-bromo-4-phénoxybenzoïque de formule 38a 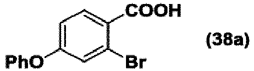
b) réaction d'un composé de formule 38a avec l'isocyanoacétate d'éthyle lors de l'activation du groupe carboxyle avec un agent d'activation approprié, de préférence le 1,1'carbonyldiimidazole, pour former l'intermédiaire de formule 39a.

6. Un procédé de préparation selon les revendications 1 et 2, **caractérisé en ce que** l'intermédiaire de formule 32 est un composé de formule 40-Al, 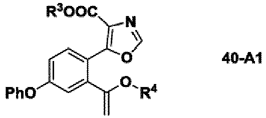 qui, dans un environnement acide, est soumis à l'ouverture du cycle oxazole, à l'hydrolyse du groupe vinyléther en groupe acétyle et à la condensation ultérieure pour donner l'intermédiaire 17, 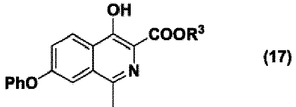 dans lesquels R³ et R⁴ sont un groupe alkyle en C₁-C₁₀.

7. Un procédé de préparation selon les revendications 1 et 2, **caractérisé en ce que** l'intermédiaire de formule 32 est un composé de formule 40-A2, 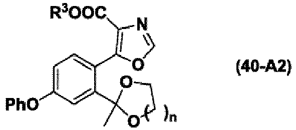 qui, en milieu acide, est soumis à l'ouverture du cycle oxazole, à l'hydrolyse du groupe dioxolanne en groupe acétyle et à sa condensation ultérieure pour donner l'intermédiaire 17, 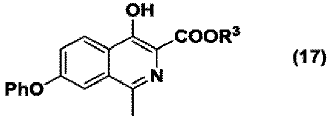 dans laquelle R³ est un groupe alkyle en C₁-C₁₀ et n = 1 à 5.

8. Un procédé de préparation selon les revendications 1 et 2, **caractérisé en ce que** l'intermédiaire de formule 32 est un composé de formule 40-A3, 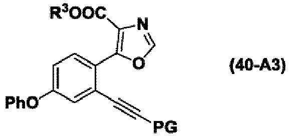 qui, dans un environnement acide, est soumis à l'ouverture du cycle oxazole, déprotection du groupe acétylénique, son hydrolyse en groupe acétyle et la condensation ultérieure pour donner le intermédiaire 17, 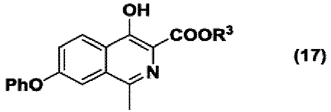 dans laquelle R³ est un groupe alkyle en C₁-C₁₀ et PG est un groupe protecteur approprié, de préférence R⁵R⁶R⁷Si, dans lequel les groupes alkyle en R⁵, R⁶ et R⁷ sont des groupes alkyle en C₁-C₁₀.

9. Un procédé de préparation selon les revendications 1 et 2 **caractérisé en ce que** l'intermédiaire de formule 32 est un composé de formule 40-A4, 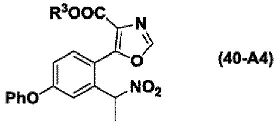 qui, dans un environnement acide, est soumis à l'ouverture du cycle oxazole, l'hydrolyse du groupe nitroalkyle en groupe acétyle et condensation ultérieure pour donner l'intermédiaire 17, 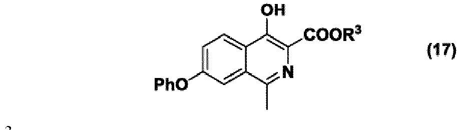 dans lequel R³ est un groupe alkyle en C₁-C₁₀.

10. Un procédé de préparation selon les revendications 6 à 9, **caractérisé en ce que** l'on convertit l'intermédiaire de formule 17, 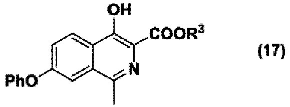 par réaction avec la glycine ou ses dérivés fonctionnels en roxadustat deformule 1,

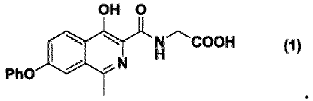

11. 5-(2-bromo-4-phénoxyphényl)oxazole-4-carboxylate d'éthyle de formule 39a.

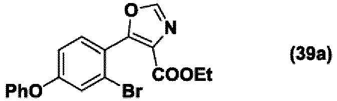

12. 3-(2-acétyl-4-phénoxyphényl)-2-amino-3-oxopropanoate d'éthyle de formule 33.

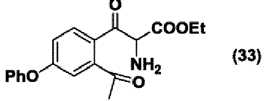

13. 5-(2-(1-butoxyvinyl)-4-phénoxyphényl)oxazole-4-carboxylate d'éthyle de formule 40-A1a.

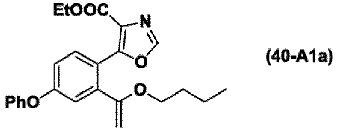

14. 5-(2-(1-éthoxyvinyl)-4-phénoxyphényl)oxazole-4-carboxylate d'éthyle de formule 40-Alb.

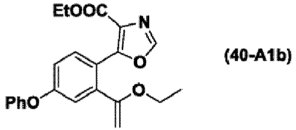

15. 5-(2-(2-méthyl-1,3-dioxolan-2-yl)-4-phénoxyphényl)oxazole-4-carboxylate d'éthyle de formule 40-A2a.

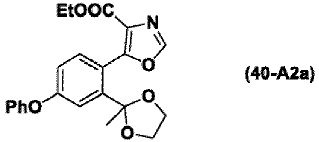

16. 5-(2-(2-méthyl-1,3-dioxépan-2-yl)-4-phénoxyphényl)oxazole-4-carboxylate d'éthyle de formule 40-A2b.

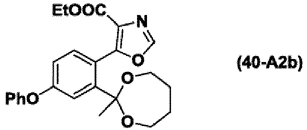

17. 5-(4-phénoxy-2-((triméthylsilyl)éthynyl)phényl)oxazole-4-carboxylate d'éthyle de formule40-A3a.

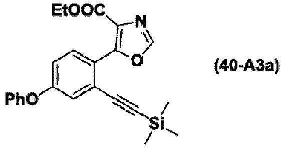

18. 5-(4-phénoxy-2-((triisopropylsilyl)éthynyl)phényl)oxazole-4-carboxylate d'éthyle de formule 40-A3b.

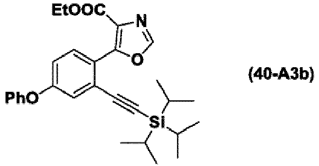

19. 5-(2-éthynyl-4-phénoxyphényl)oxazole-4-carboxylate d'éthyle de formule 40-42a.
